# EUROPEAN PATENT APPLICATION

(11) **EP 4 400 496 A1**
(43) Date of publication of application: **17.07.2024**
(21) Application number: 22867314.1
(22) Date of filing: 05.09.2022
(51) Int. Cl.: C07D 307/68, B01J 27/185, C07B 61/00

(54) **METHOD FOR PRODUCING COMPOUND**

(30) Priority: 07.09.2021 JP 2021145084
(71) Applicant: MITSUBISHI GAS CHEMICAL COMPANY, INC., Chiyoda-ku Tokyo 100-8324 (JP)
(72) Inventor: KIRINO, Tomoaki, Tokyo 100-8324 (JP); OHNO, Daisuke, Tokyo 100-8324 (JP); NAKAJIMA, Kiyotaka, Sapporo-shi, Hokkaido 060-0808 (JP); FUKUOKA, Atsushi, Sapporo-shi, Hokkaido 060-0808 (JP)
(74) Representative: Müller-Boré & Partner Patentanwälte PartG mbB
(86) International application number: PCT/JP2022/033205
(87) International publication number: WO 2023/037992

(57) **Abstract**

Provided is a method for producing an aminomethylcarboxylic acid compound by subjecting a compound containing a formyl group and a carboxyl group to amination while generation of byproducts is effectively suppressed. The method for producing a compound represented by Formula (2) includes reacting a compound represented by Formula (1) and an ammonium salt:

Formula (1) HOOC-X¹-C(=O)H

where in Formula (1) X¹ is a divalent organic group; and

Formula (2) HOOC-X¹-CH₂NH₂

where in Formula (2) X¹ is a divalent organic group.

## Description

### Technical Field

The present invention relates to a method for producing a compound. The present invention particularly relates to a method for reductive amination of a formyl group in a carboxylic acid containing a formyl group such as formylfurancarboxylic acid.

### Background Art

Amination of a compound containing a formyl group by using a nano-cobalt catalyst has been studied. For example, Non-Patent Literature 1 describes the following reaction mechanism.

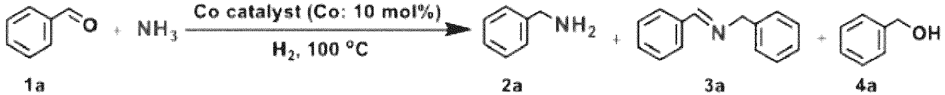

### Citation List

### Non-Patent Literatures

[Non-Patent Literature 1] JACS Au 2021, 1, 501-507

### Summary of Invention

### Technical Problem

When the inventors of the present invention conducted studies, it was found that, in reacting ammonia with the compound containing a formyl group described in Non-Patent Literature 1 in the presence of a cobalt catalyst, the formyl group is aminated; however, in a case where the compound containing a formyl group contains a carboxyl group, the amination does not sufficiently proceed, and a large amount of byproducts are generated.

The present invention is to solve the issue described above, and an object of the present invention is to provide a method for producing an aminomethylcarboxylic acid compound by subjecting a compound containing a formyl group and a carboxyl group to amination while generation of byproducts is effectively suppressed.

### Solution to Problem

For such an object, the issue described above has been discovered to be resolved by using an ammonium salt in place of ammonia water as a nitrogen source for the amination. Specifically, the issue described above is solved by the following solutions.
<1> A method for producing a compound represented by Formula (2), the method including reacting a compound represented by Formula (1) and an ammonium salt:

   Formula (1) HOOC-X¹-C(=O)H

   where in Formula (1), X¹ is a divalent organic group,

   Formula (2) HOOC-X¹-CH₂NH₂

   where in Formula (2), X¹ is a divalent organic group.
<2> The production method according to <1>, where X¹ in Formula (1) and Formula (2) has an aromatic ring.
<3> The production method according to <1>, where X¹ in Formula (1) and Formula (2) has a furan ring.
<4> The production method according to any one of <1> to <3>, where the compound represented by Formula (1) is a compound represented by Formula (1-1), and the compound represented by Formula (2) is a compound represented by Formula (2-1): 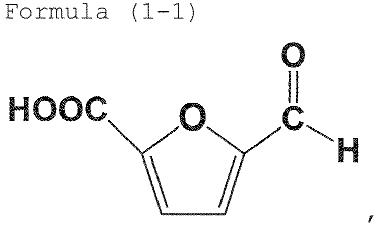 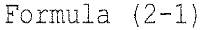 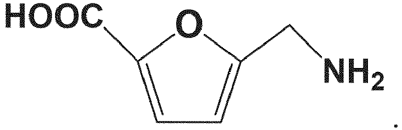
<5> The production method according to any one of <1> to <4>, where the ammonium salt is an ammonium salt of an organic acid.
<6> The production method according to any one of <1> to <4>, where the ammonium salt contains at least one selected from the group consisting of ammonium carbonate, ammonium formate, ammonium acetate, ammonium propionate, and ammonium butyrate.
<7> The production method according to any one of <1> to <4>, where the ammonium salt contains ammonium acetate.
<8> The production method according to any one of <1> to <7>, where the reaction is performed in a presence of cobalt phosphide.
<9> The production method according to any one of <1> to <8>, where the reaction is performed in a presence of a water-containing solvent.
<10> The production method according to any one of <1> to <9>, where the reaction is performed in a presence of a solvent containing a compound represented by A-(OH)m, where A is a hydrocarbon group having from 1 to 10 carbons, and m is 1 or 2.
<11> The production method according to <10>, where m in the compound represented by A-(OH)m is 1.
<12> The production method according to <10>, where the compound represented by A-(OH)m contains methanol.
<13> The production method according to any one of <1> to <12>, where the reaction is performed in a presence of a solvent containing water and a compound represented by A-(OH)m, where A is a hydrocarbon group having from 1 to 10 carbons, and m is 1 or 2; and a volume ratio between the water and the compound represented by A-(OH)m is greater than 0 and 10 or less with respect to 1 water.
<14> The production method according to any one of <1> to <13>, where the reaction is performed in a presence of a primary alkylamine.
<15> The production method according to <14>, where the primary alkylamine is n-butylamine.
<16> The production method according to any one of <1> to <15>, where the production method produces a compound represented by Formula (3) together with the compound represented by Formula (2):

   Formula (3) HOOC-X¹-CH₂OH

   where in Formula (3), X¹ is a divalent organic group.
<17> The production method according to <16>, where the compound represented by Formula (3) is a compound represented by Formula (3-1): 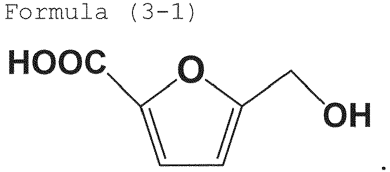
<18> A composition containing a compound represented by Formula (2-1) and a compound represented by Formula (3-1):

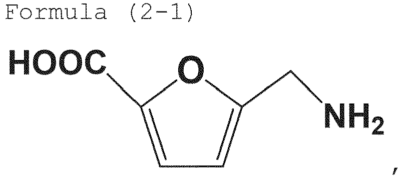

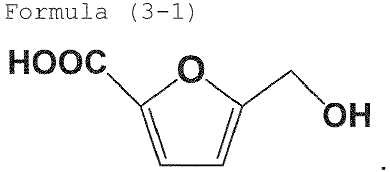

### Advantageous Effects of Invention

The present invention can provide a method for producing an aminomethylcarboxylic acid compound by subjecting a compound containing a formyl group and a carboxyl group to amination while generation of byproducts is effectively suppressed.

### Description of Embodiments

Hereinafter, embodiments for conducting the present invention (referred to simply as "the present embodiment" below) will be described in detail. Note that the present embodiment below is an example for describing the present invention, and the present invention is not limited to the present embodiment.

In the present specification, "from ... to ..." or "of ... to ..." is used to mean that the numerical values described before and after "to" are included as the lower limit and the upper limit, respectively.

In the present specification, various physical property values and characteristic values are values at 23°C unless otherwise noted.

In a description of a group (atomic group) in the present specification, a description not specifying whether the group is a substituted group or an unsubstituted group is meant to include a group (atomic group) having a substituent as well as a group (atomic group) having no substituent. For example, an "alkyl group" includes not only an alkyl group having no substituent (unsubstituted alkyl group) but also an alkyl group having a substituent (substituted alkyl group). In the present specification, a description not specifying whether the group is a substituted group or an unsubstituted group means that the group is preferably an unsubstituted group.

When a measurement method or the like of a standard set forth in the present specification differs depending on the year, it is based on the standard as of January 1, 2021, unless otherwise stated.

The method for producing a compound of the present embodiment is a method for producing a compound represented by Formula (2), the method including reacting a compound represented by Formula (1) and an ammonium salt:

Formula (1) HOOC-X¹-C(=O)H

where in Formula (1), X¹ is a divalent organic group,

Formula (2) HOOC-X¹-CH₂NH₂

where in Formula (2), X¹ is a divalent organic group.

For the compound having a carboxyl group (-COOH) and a formyl group (-C(=O)H), when ammonia water is reacted to aminate the formyl group, byproducts are generated. In particular, when the byproducts are substances other than hydroxymethylcarboxylic acid, there are many limitations in use for various purposes, and purification is required. In the present embodiment, this issue was solved by using an ammonium salt in place of the ammonia water during amination of the formyl group. That is, when the inventors of the present invention studied, it was found that, when ammonia water is reacted with the compound having a carboxyl group, a side reaction of the formyl group occurs, and byproducts tend to be generated. In the present embodiment, it is presumed that generation of such byproducts can be effectively suppressed by the use of the ammonium salt.

In the present embodiment, the compound represented by Formula (1) is a compound serving as a starting material of the reaction in the present embodiment. The compound represented by Formula (1) is typically charged in a reaction system but may be an intermediate product obtained by using another compound as a starting material:

Formula (1) HOOC-X^{x}-C(=O)H

where in Formula (1), X¹ is a divalent organic group.

In the present embodiment, X¹ in Formula (1) is a divalent organic group and is preferably at least one of an aliphatic group, an aromatic group, -O-, -NH-, or -S-, or is preferably a group made of a combination of two or more of the foregoing. However, a group that is adjacent to -COOH and -C(=O)H in Formula (1) is preferably an aromatic group or an aliphatic group. Furthermore, the aromatic group and the aliphatic group may contain a substituent (substituent A) described below or not contain the substituent.

More specifically, X¹ preferably contains an alicycle or an aromatic ring, and more preferably contains an aromatic ring. The aromatic ring may be an aromatic hydrocarbon ring or an aromatic heterocycle and is preferably an aromatic heterocycle. The aromatic ring is preferably a 5-membered ring or a 6-membered ring, and more preferably a 5-membered ring. Furthermore, the aromatic ring may be a monocycle or a condensed ring and is preferably a monocycle. Specifically, a benzene ring is preferred as the aromatic hydrocarbon ring. Examples of the aromatic heterocycle include a furan ring, a thiophene ring, a pyrrole ring, a pyrazole ring, an imidazole ring, an oxazole ring, a thiazole ring, a pyridine ring, a pyrimidine ring, and a pyrane, and a furan ring is preferred.

Furthermore, X¹ may consist only of an aromatic ring or may contain a linking group that links groups (-COOH and -C(=O)H) adjacent to the aromatic ring or may contain a substituent of an aromatic ring. In the present embodiment, in X¹, the aromatic ring preferably directly bonds to the adjacent groups (-COOH and -C(=O)H). Examples of the substituent (substituent A) include an alkyl group having from 1 to 10 carbons, an alkyloxy group having from 1 to 10 carbons, an alkylthio group having from 1 to 10 carbons, an aryl group having from 6 to 10 carbons, an aryloxy group having from 1 to 10 carbons, an arylthio group having from 1 to 10 carbons, a halogen atom, a hydroxy group, or a mercapto group. The substituent (substituent A) is preferably an alkyl group having from 1 to 10 carbons, an alkyloxy group having from 1 to 10 carbons, a halogen atom, or a hydroxy group. The number of the substituent is preferably from 0 to 3, and more preferably from 0 to 2.

In addition, in the present embodiment, an alicycle or an aliphatic hetero cycle, such as a cyclohexane ring or a tetrahydrofuran ring, is preferably contained. These rings may also contain a substituent (substituent A) or a linking group but preferably do not contain these.

The molecular weight of the compound represented by Formula (1) is preferably 125 or greater and preferably 500 or less.

In the present embodiment, the compound represented by Formula (2) is a compound that is a product. Typically, the compound represented by Formula (2) is taken out from the reaction system. However, the compound represented by Formula (2) may be an intermediate product for producing another compound:

Formula (2) HOOC-X¹-CH₂NH₂

where in Formula (2), X¹ is a divalent organic group.

X¹ in Formula (2) is the same as that in Formula (1).

In the present embodiment, the compound represented by Formula (1) is preferably a compound represented by Formula (1-1). Furthermore, the compound represented by Formula (2) is preferably a compound represented by Formula (2-1):

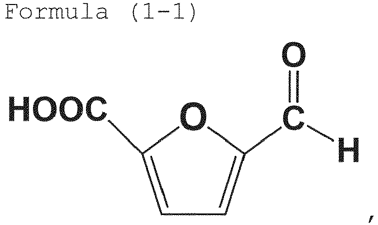

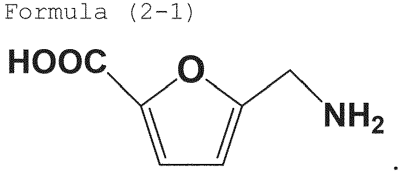

In the production method of the present embodiment, a compound represented by Formula (3) may be produced together with the compound represented by Formula (2). However, the product preferably contains the compound represented by Formula (2) but preferably contains no compound represented by Formula (3) :

Formula (3) HOOC-X¹-CH₂OH

where in Formula (3), X¹ is a divalent organic group.

X¹ in Formula (3) is the same as that in Formula (1). Furthermore, the compound represented by Formula (3) is preferably a compound represented by Formula (3-1).

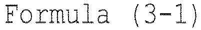

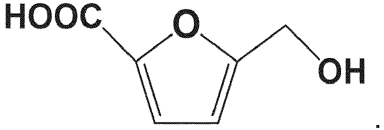

In a case where the product obtained in the production method of the present embodiment is a composition containing the compound represented by Formula (2) and the compound represented by Formula (3), the mass ratio (Formula (2):Formula (3)) is preferably from 99.9:0.1 to 50:50, more preferably from 99.9:0.1 to 60:40, even more preferably from 99.9:0.1 to 70:30, and yet even more preferably from 99.9:0.1 to 80:20.

In the present embodiment, the compound represented by Formula (1) and an ammonium salt are reacted. By allowing the ammonium salt to be reacted, progression of a side reaction of the formyl group can be effectively suppressed.

As the ammonium salt, a salt obtained by neutralizing an organic acid and an inorganic acid in ammonia can be used. Examples of the ammonium salt of an organic acid include ammonium carbonate, ammonium formate, ammonium acetate, ammonium propionate, ammonium butyrate, ammonium isobutyrate, ammonium oxalate, ammonium succinate, ammonium adipate, ammonium benzoate, and diammonium phthalate. Examples of the ammonium salt of an inorganic acid include ammonium phosphate, monoammonium phosphate, diammonium phosphate, ammonium borate, ammonium pentaborate, and ammonium tetraborate. Among these, an ammonium salt of an organic acid is preferred. An ammonium salt containing at least one selected from the group consisting of ammonium carbonate, ammonium formate, ammonium acetate, ammonium propionate, and ammonium butyrate is more preferred, and an ammonium salt containing ammonium acetate is even more preferred.

In the production method of the present embodiment, the nitrogen source amount is preferably 1 mol or greater and may be 4 mol or greater, or 8 mol or greater, with respect to the amount of the raw material (1 mol of formyl group contained in the compound represented by Formula (1)). Furthermore, the nitrogen source amount is preferably 50 mol or less and may be 40 mol or less, 30 mol or less, 20 mol or less, or 15 mol or less, with respect to 1 mol of the raw material (compound represented by Formula (1)).

In the production method of the present embodiment, one of the ammonium salt may be used, or two or more of the ammonium salts may be used. When two or more are used, the total amount is preferably within the range described above.

In the production method of the present embodiment, reaction is preferably progressed in the presence of a solvent.

In the production method of the present embodiment, the mass ratio (raw material concentration) of the compound represented by Formula (1) to the solvent amount is preferably 0.1 mass% or greater and may be 0.5 mass% or greater, 1.0 mass% or greater, 1.5 mass% or greater, or 2.0 mass% or greater. When the mass ratio is not lower than the lower limit described above, the reaction tends to be faster. The upper limit of the raw material concentration is not particularly limited; however, because the raw material may not be dissolved when the raw material concentration is large, the raw material concentration is preferably 50 mass% or less and may be 30 mass% or less, 25 mass% or less, 20 mass% or less, 15 mass% or less, or 9 mass% or less.

In the production method of the present embodiment, one kind of the compound represented by Formula (1), which is a raw material, may be used, or two or more kinds of the compounds represented by Formula (1) may be used; however, using one kind of the compound represented by Formula (1) is preferable. When two or more kinds are used, the total amount is preferably within the range described above.

In the production method of the present embodiment, reaction is preferably progressed in the presence of a water-containing solvent. It is presumed that, because the solvent contains water, the ammonium salt is dissolved in the water and the reaction is promoted.

In the production method of the present embodiment, the reaction is preferably progressed in the presence of a solvent containing a compound represented by A-(OH)m (A is a hydrocarbon group having from 1 to 10 carbons, and m is 1 or 2). By using such a solvent, generation of byproducts can be effectively suppressed. That is, it is presumed that, because the formyl group is protected by the compound represented by A-(OH)m, progression of a side reaction of the formyl group can be effectively suppressed.

In the compound represented by A-(OH)m, A is preferably a hydrocarbon group having from 1 to 10 carbons, more preferably a hydrocarbon group having from 1 to 5 carbons, and even more preferably a hydrocarbon group having from 1 to 3 carbons. Furthermore, the hydrocarbon group is preferably an alkyl group.

In the present embodiment, when m is 1, A is preferably a methyl group or an ethyl group, and more preferably a methyl group.

In the present embodiment, when m is 2, A is preferably an ethylene group, a propylene group, or a butylene group.

In the compound represented by A-(OH)m, m is preferably 1.

Specific examples of the compound represented by A-(OH)m include an alkyl monoalcohol, such as methanol, ethanol, n-propanol, isopropanol, n-butanol, sec-butanol, isobutanol, tert-butanol, 3-pentanol, 2-methyl-1-butanol, 3-methyl-1-butanol, 2-methyl-2-butanol, 3-methyl-2-butanol, and neopentyl alcohol; and an alkylene diol, such as ethylene glycol, 1,3-propane diol, and 1,4-butane diol. An alkyl monoalcohol is preferred, methanol and ethanol are more preferred, and methanol is even more preferred.

The solvent used in the present embodiment preferably contains the compound represented by A-(OH)m and water. By using a combination of water and the compound represented by A-(OH)m as a solvent, generation of the compound of Formula (3) can be effectively suppressed.

In the present embodiment, in a case where a combination of water and the compound represented by A-(OH)m as a solvent is used, the volume ratio between the water and the compound represented by A-(OH)m is preferably greater than 0 and may be 0.1 or greater, greater than 1.0, or 1.5 or greater, with respect to 1 water. Furthermore, the volume ratio between the water and the compound represented by A-(OH)m is preferably 10 or less, and more preferably 5 or less, and may be 3 or less, with respect to 1 water.

In the production method of the present embodiment, one kind of the compound represented by A-(OH)m may be used, or two or more kind of the compounds represented by A-(OH)m may be used. When two or more kind are used, the total amount is preferably within the range described above.

In the solvent used in the present embodiment, the total amount of the compound represented by A-(OH)m and the water (however, this does not mean that these components are necessarily contained) preferably accounts for 90 vol% or greater, more preferably 95 vol% or greater, and even more preferably 99 vol% or greater, of the solvent. Note that, in the present embodiment, a primary alkylamine is not treated as a solvent but as an additive.

In the production method of the present embodiment, the amination reaction is preferably performed in the presence of a primary alkylamine. By addition of the primary alkylamine, generation of the compound represented by Formula (3) can be effectively suppressed by protecting the formyl group. That is, as described below, imination is promoted by the primary alkylamine (e.g., n-butylamine (BA)), and a side reaction (generation of the compound represented by Formula (3)) can be effectively suppressed.

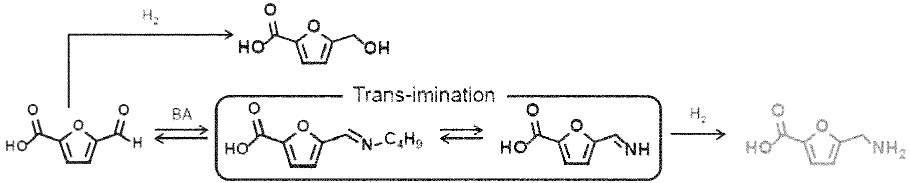

As the primary alkylamine, a primary alkylamine having from 1 to 10 carbons is preferred, methylamine, ethylamine, n-propylamine, isopropylamine, n-butylamine, t-butylamine, isobutylamine, n-pentylamine, t-pentylamine, isopentylamine, 2-methylbutylamine, n-hexylamine, and n-heptylamine are more preferred, methylamine, ethylamine, n-propylamine, isopropylamine, n-butylamine, t-butylamine, and isobutylamine are even more preferred, and n-butylamine is yet even more preferred.

In a case where the primary alkylamine is blended in the production method of the present embodiment, the blended amount thereof is preferably 0.1 mol or greater, and more preferably 0.4 mol or greater, with respect to 1 mol of the formyl group of the raw material (compound represented by Formula (1)). When the blended amount is not lower than the lower limit described above, effect of protecting the formyl group tends to further improve. Furthermore, the amount of the primary alkylamine is preferably less than 10 mol, more preferably 5 mol or less, and even more preferably 2 mol or less, with respect to 1 mol of the formyl group of the raw material (compound represented by Formula (1)).

In a case where the primary alkylamine is blended in the production method of the present embodiment, the blended amount thereof is preferably 1 mol or less, and more preferably 0.5 mol or less, and may be 0.3 mol or less, with respect to 1 mol of the ammonium salt. When the blended amount is not higher than the upper limit described above, exchange reaction between the imine compound protected by the primary alkylamine and the ammonium salt (trans-imination described above) tends to be effectively promoted. The lower limit is not particularly limited and, for example, practically 0.01 mol or greater with respect to 1 mol of the ammonium salt.

In the production method of the present embodiment, one kind of primary alkylamine may be used, or two or more kinds of the primary alkylamines may be used. When two or more kinds are used, the total amount is preferably within the range described above.

In the production method of the present embodiment, the reaction may be promoted by using a catalyst. As a catalyst, its kind and the like are not particularly limited, but the catalyst is preferably metal nickel, metal cobalt, a nickel compound, or a cobalt compound, and for example, more preferably nickel, cobalt, nickel phosphide, or cobalt phosphide. Among these, cobalt phosphide is preferred, and particles of cobalt phosphide are more preferred. For other details of the cobalt phosphide, refer to the disclosures of JP 2021-013923 A, the contents of which are incorporated herein by reference.

The catalyst amount in the production method of the present embodiment is, in a case where a catalyst is used, preferably 0.01 mol or greater and may be 0.05 mol or greater, or 0.08 mol or greater, with respect to 1 mol of the formyl group of the raw material (compound represented by Formula (1)). Furthermore, the catalyst amount may be 10 mol or less, 5 mol or less, 3 mol or less, 0.4 mol or less, or 0.2 mol or less, with respect to 1 mol of the raw material (compound represented by Formula (1)).

In the production method of the present embodiment, one kind of the catalyst may be used, or two or more kinds of the catalysts may be used. When two or more kinds are used, the total amount is preferably within the range described above.

The production method of the present embodiment is preferably performed under increased pressure by hydrogen.

The increased pressure by hydrogen is preferably 1 bar or greater and preferably 10 bar or less.

The reaction temperature of the amination reaction in the production method of the present embodiment is preferably 50°C or higher, more preferably 90°C or higher, even more preferably 95°C or higher, yet even more preferably 100°C or higher, and yet even more preferably 105°C or higher. Furthermore, the reaction temperature of the amination reaction is preferably 200°C or lower, more preferably 150°C or lower, even more preferably 140°C or lower, yet even more preferably 130°C or lower, and yet even more preferably 125°C or lower.

In the production method of the present embodiment, the reaction temperature may be a constant temperature (however, change in ±5°C is taken as a margin of error) besides temperatures at initial temperature elevation and temperature decrease at the end, or may be reacted in a two- or more-step reaction. In the present embodiment, the reaction temperature is preferably a constant temperature (however, change in ±5°C is taken as a margin of error) besides temperatures at initial temperature elevation and temperature decrease at the end.

The reaction time for the amination reaction in the production method of the present embodiment is preferably 1 minute or longer and may be 30 minutes or longer, 1 hour or longer, or 2 hours or longer. Furthermore, the reaction time of the amination reaction may be 50 hours or shorter, 30 hours or shorter, or 20 hours or shorter.

The separation of the reaction mixture from the catalyst after the reaction can be performed by a typical method, such as precipitation, centrifugation, or filtration. Depending on the catalyst used, appropriately, the separation of the catalyst is preferably performed in an inert gas atmosphere, such as nitrogen or argon, to prevent ignition. Furthermore, the obtained reaction solution may be optionally concentrated, and then the residue may be used as is as a raw material or an intermediate product, or the reaction mixture may be appropriately post-treated and purified. Specific examples of the method of the post-treatment include well-known purification methods, such as extraction, distillation, and chromatography. A combination of two or more kinds of these purification methods may be performed.

In the production method of the present embodiment, a higher conversion ratio of the raw material is more preferred, and the conversion ratio is preferably 50 mol% or greater, and more preferably 80 mol% or greater. The upper limit is ideally 100 mol%.

In the production method of the present embodiment, a higher yield of the compound represented by Formula (2) is more preferred, and the yield is preferably 30% or greater, more preferably 50% or greater, and even more preferably 70% or greater. The upper limit is ideally 100 mol%.

In the production method of the present embodiment, a lower yield of byproducts other than the compound represented by Formula (3) is more preferred, and the yield is more preferably 40% or less, even more preferably 30% or less, yet even more preferably 20% or less, and yet even more preferably 10% or less. The lower limit is ideally 0%.

The compound represented by Formula (2) obtained in the present embodiment and a composition containing the compound represented by Formula (2) and the compound represented by Formula (3) are preferably used as various industrial materials.

### Examples

The present invention will be described more specifically with reference to examples below. Materials, amounts used, proportions, processing details, processing procedures, and the like described in the following examples can be appropriately changed as long as they do not depart from the spirit of the present invention. Thus, the scope of the present invention is not limited to the specific examples described below.

If a measuring device used in the examples is not readily available due to discontinuation or the like, another device with equivalent performance can be used for measurement.

### Synthesis Example of Cobalt Phosphide Catalyst

Cobalt chloride (CoCl₂) (1.0 mmol), hexadecylamine (10 mmol), triphenyl phosphite (10 mmol), 1-octadecene (10.0 mL) were added in a Schlenk flask and agitated. The liquid mixture was heated in an argon stream at 150°C for 1 hour. Then, the temperature was increased to a solvent boiling point (approximately 290°C) in 20 minutes and then maintained for 2 hours. Thereafter, the temperature was decreased to 200°C and then rapidly decreased to room temperature in a water bath, and thus a black product was obtained. The obtained black product was washed with acetone, precipitated, recovered, further washed using chloroform and acetone, and dried in the air, and thus a cobalt phosphide catalyst was obtained.

### Example 1

In an autoclave, 0.5 mmol of raw material (formylfurancarboxylic acid (FFCA)), water (in an amount that made the raw material concentration 2.5 mass%), 0.05 mmol of the cobalt phosphide catalyst obtained as described above, and 5 mmol of nitrogen source (ammonium acetate) were charged and reacted at a hydrogen pressure of 5 bar, at a reaction temperature of 120°C for 16 hours.

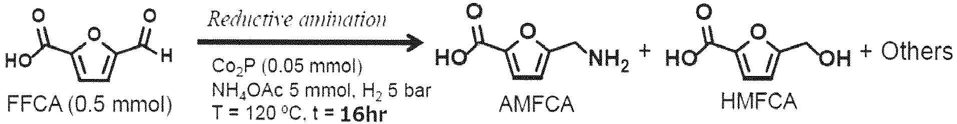

The conversion ratio was calculated by quantification of the raw material by high performance liquid chromatography (HPLC) analysis.

The conversion ratio was expressed in units of %. The results are indicated in Table 1.

The HPLC analysis of the reaction solution was performed, and identification and quantification of the raw material and the product were performed. The amount of generated byproducts that were not detected by the HPLC analysis was calculated based on the amounts of the raw material and the product.

The each component of the product was expressed in units of %. The results are indicated in Table 1.

### Examples 2 to 4 and Reference Example 1

Changes from Example 1 were made as indicated in Table 1, and others were performed in the same manner. Note that methanol and/or n-butylamine were charged into the reaction system at the same time as an aqueous solution of 0.5 mmol formylfurancarboxylic acid (FFCA).

Note that, in Table 1, MeOH means methanol, NH₄OA_{C} means ammonium acetate, and NH₃aq means ammonia water.

**[Table 1]**

| | Example 1 | Example 2 | Reference Example 1 | Example 3 | Example 4 |
|---|---|---|---|---|---|
| Raw material (mmol) | 0.5 | 0.5 | 0.5 | 0.5 | 2 |
| Raw material concentration (mass%) | 2.5 | 2.5 | 2.5 | 2.5 | 10 |
| Catalyst amount (mmol) | 0.05 | 0.05 | 0.05 | 0.05 | 0.2 |
| Solvent | H₂O | H₂O | H₂O | H₂O/MeOH | H₂O/MeOH |
| Solvent ratio (vol/vol) | | | | 1/2 | 1/2 |
| N source | NH₄OAc | NH₄OAc | NH₃aq | NH₄OAc | NH₄OAc |
| N source amount (mmol) | 5 | 5 | 5 | 5 | 5 |
| Butylamine (mmol) | | | | | 1 |
| H₂ pressure (bar) | 5 | 5 | 5 | 5 | 5 |
| Temperature (°C) | 120 | 120 | 120 | 120 | 120 |
| Reaction time (hr) | 16 | 3 | 3 | 3 | 3 |
| | | | | | |
| Conversion rate | 94 | 59 | 98 | 100 | 90 |
| AMFCA | 55 | 35 | 55 | 95 | 73 |
| HMFCA | 20 | 12 | 6 | 2 | 10 |
| Byproduct | 19 | 12 | 37 | 3 | 7 |

The solvent ratio (vol/vol) described above indicates a volume ratio of water (H₂O) and methanol (MeOH).

AMFCA means aminomethylfurancarboxylic acid, and HMFCA means hydroxymethylfurancarboxylic acid.

As is clear from the results described above, with the production method of the present embodiment, generation of byproducts was effectively suppressed. Furthermore, generation of byproducts can be further reduced by blending alcohol in addition to water as the solvent.

## Claims

1. A method for producing a compound represented by Formula (2), the method comprising reacting a compound represented by Formula (1) and an ammonium salt:
Formula (1) HOOC-X¹-C(=O)H
where in Formula (1), X¹ is a divalent organic group; and
Formula (2) HOOC-X¹-CH₂NH₂
where in Formula (2), X¹ is a divalent organic group.

2. The production method according to claim 1, wherein X¹ in Formula (1) and Formula (2) has an aromatic ring.

3. The production method according to claim 1, wherein X¹ in Formula (1) and Formula (2) has a furan ring.

4. The production method according to any one of claims 1 to 3, wherein the compound represented by Formula (1) is a compound represented by Formula (1-1), and the compound represented by Formula (2) is a compound represented by Formula (2-1):

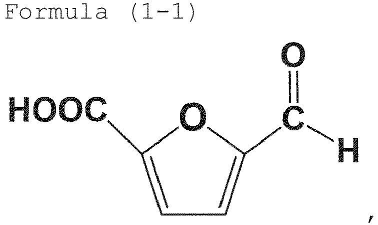

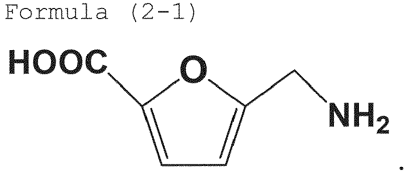

5. The production method according to any one of claims 1 to 4, wherein the ammonium salt is an ammonium salt of an organic acid.

6. The production method according to any one of claims 1 to 4, wherein the ammonium salt comprises at least one selected from the group consisting of ammonium carbonate, ammonium formate, ammonium acetate, ammonium propionate, and ammonium butyrate.

7. The production method according to any one of claims 1 to 4, wherein the ammonium salt comprises ammonium acetate.

8. The production method according to any one of claims 1 to 7, wherein the reaction is performed in a presence of cobalt phosphide.

9. The production method according to any one of claims 1 to 8, wherein the reaction is performed in a presence of a water-containing solvent.

10. The production method according to any one of claims 1 to 9, wherein the reaction is performed in a presence of a solvent containing a compound represented by A-(OH)m, where A is a hydrocarbon group having from 1 to 10 carbons, and m is 1 or 2.

11. The production method according to claim 10, wherein m in the compound represented by A-(OH)m is 1.

12. The production method according to claim 10, wherein the compound represented by A-(OH)m comprises methanol.

13. The production method according to any one of claims 1 to 12, wherein the reaction is performed in a presence of a solvent containing water and a compound represented by A-(OH)m, where A is a hydrocarbon group having from 1 to 10 carbons, and m is 1 or 2; and a volume ratio between the water and the compound represented by A-(OH)m is greater than 0 and 10 or less with respect to 1 water.

14. The production method according to any one of claims 1 to 13, wherein the reaction is performed in a presence of a primary alkylamine.

15. The production method according to claim 14, wherein the primary alkylamine is n-butylamine.
